**Europäisches Patentamt**

**European Patent Office**

**Office européen des brevets**

(19)

(11) Veröffentlichungsnummer: **0 218 789**
**B1**

(12) **EUROPÄISCHE PATENTSCHRIFT**

(45) Veröffentlichungstag der Patentschrift:
29.11.89

(21) Anmeldenummer: 86107944.0

(22) Anmeldetag: 11.06.86

(51) Int. Cl.⁴: **A61N 1/36**

(54) Vorhofgesteuerter Herzschrittmacher.

(30) Priorität: 04.10.85 DE 3535568

(43) Veröffentlichungstag der Anmeldung:
22.04.87 Patentblatt 87/17

(45) Bekanntmachung des Hinweises auf die Patenterteilung:
29.11.89 Patentblatt 89/48

(84) Benannte Vertragsstaaten:
DE FR GB IT NL SE

(56) Entgegenhaltungen:
EP-A- 0 081 209
EP-A- 0 108 360
GB-A- 2 073 023
GB-A- 2 076 655
GB-A- 2 116 845
GB-A- 2 142 539

(73) Patentinhaber: Siemens Aktiengesellschaft,
Wittelsbacherplatz 2, D-8000 München 2(DE)
(84) Benannte Vertragsstaaten: DE FR GB IT NL

(73) Patentinhaber: Siemens Elema AB, Röntgenvägen 2,
S-171 95 Solna 1(SE)
(84) Benannte Vertragsstaaten: SE

(72) Erfinder: Hedberg, Sven-Erik, Dipl.-Ing., Odonstigen 5,
S-196 31 Kungsängen(SE)
Erfinder: Lindgren, Anders, Dipl.-Ing., Sportvägen 24,
S-183 40 Täby(SE)
Erfinder: Lekholm, Anders, Dr., Gnejsvägen 4,
S-161 39 Bromma(SE)

**Beschreibung**

Bei einem vorhofgesteuerten Herzschrittmacher erfolgt die Stimulation des Herzens mit veränderlicher Impulsfolgefrequenz, die vom Auftreten von Vorhofsignalen abhängt. Die Impulsfolgefrequenz kann dabei beispielsweise zwischen 50 und 150 Impulsen pro Minute variieren.

Der Erfindung liegt die Aufgabe zugrunde, einen vorhofgesteuerten Herzschrittmacher so auszubilden, daß bei dem sogenannten Wenckebach-Betrieb, bei dem der Abstand zwischen zwei ventrikulären Stimulationsimpulsen oder zwischen einer natürlichen Aktivität (QRS-Komplex) und einem Stimulationsimpuls kontinuierlich verändert wird, physiologisch ungünstige Zeitabstände zwischen Vorhof- und Ventrikelaktivitäten vermieden sind.

Diese Aufgabe ist erfindungsgemäß dadurch gelöst, daß ein kleinstes synchrones Intervall (SSI) zwischen zwei ventrikulären Stimulationsimpulsen oder zwischen einer natürlichen Aktivität und einem Stimulationsimpuls, sowie eine minimale und eine maximale atrio-ventrikuläre Verzögerungszeit AVmin, AVmax vorgegeben sind, und daß eine Steuerschaltung vorhanden ist, die in Abhängigkeit vom Auftreten eines Vorhofsignales bewirkt, daß der Herzschrittmacher mit veränderlicher Impulsrate arbeitet (DDD-Betrieb), wenn das Vorhofsignal nach dem Zeitpunkt SSI - AVmin auftritt, daß der Herzschrittmacher mit der höchsten synchronen Impulsrate betrieben wird, wenn das Vorhofsignal zwischen diesem Zeitpunkt (SSI - AVmin) und dem Zeitpunkt SSI -AVmax liegt und daß keine Stimulation erfolgt, wenn das Vorhofsignal vor dem zuletzt genannten Zeitpunkt (SSI - AVmax) auftritt.

Bei dem erfindungsgemäßen Herzschrittmacher liegt eine Wenckebach-Funktion vor, bei der die AV-Verzögerung innerhalb eines engen, definierten Bereiches variiert wird, während bei dem allgemein angewandten Wenckebach-Betrieb nur eine Verlängerung der AV-Verzögerung vorgesehen ist. Diese Verlängerung kann so groß werden, daß der AV-Synchronismus während des Wenckebach-Betriebes verlorengeht.

Die vorgeschlagene Wenckebach-Funktion nach der Erfindung ist universell. Falls AVmin gleich der normalen AV-Verzögerung gesetzt wird, geht die erfindungsgemäße Wenckebach-Funktion in eine normale Wenckebach-Funktion über, wobei jedoch eine maximale Verzögerung vorhanden ist.

Die Erfindung ist nachfolgend anhand eines in der Zeichnung dargestellten Ausführungsbeispieles näher erläutert. Es zeigen:

Fig. 1 Impulsverläufe zur Erläuterung des Erfindungsgedankens, und

Fig. 2 das Schaltbild eines Herzschrittmachers nach der Erfindung.

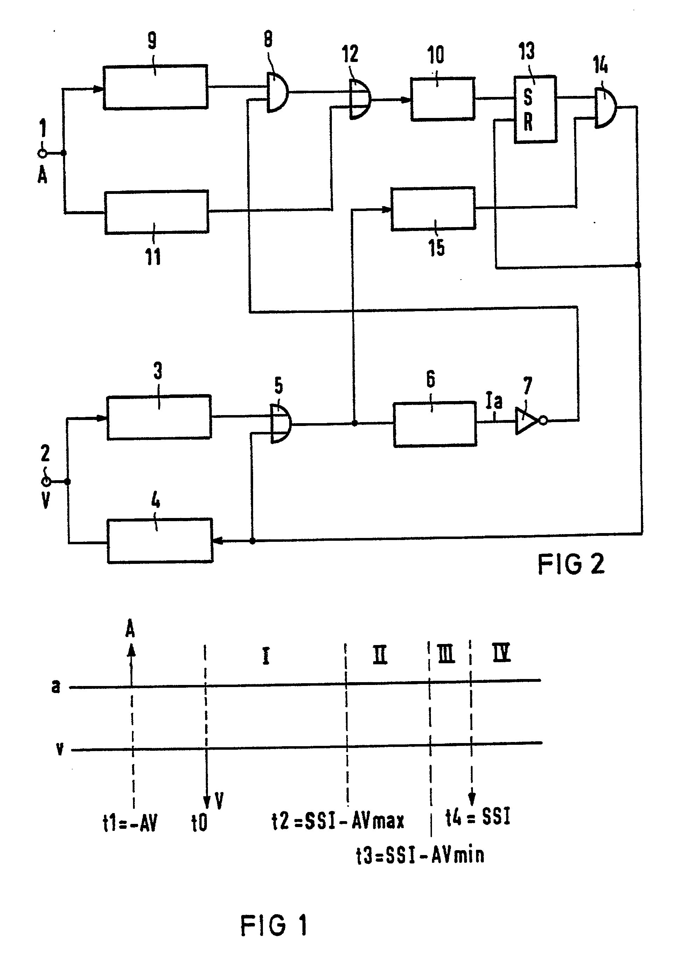

In der Figur 1 ist die Achse a die Zeitachse für die Vorhofsignale A und die Achse v die Zeitachse für die Stimulationsimpulse V. Das Vorhofsignal A tritt im Zeitpunkt t1 = -AV auf, wenn der Stimulationsimpuls V in Zeitpunkt tO auftritt. Die Zeit zwischen dem Vorhofsignal A und dem Stimulationsimpuls V ist demgemäß die AV-Verzögerung. Der Herzschrittmacher weist eine minimale AV-Verzögerung AVmin und eine maximale AV-Verzögerung AVmax auf. Ferner besitzt er eine höchste synchrone Impulsrate HSI, die das kleinste synchrone Intervall SSI zwischen zwei ventrikulären Stimulationsimpulsen oder einer natürlichen Aktivität und einem ventrikulären Stimulationsimpuls festlegt. Dieses kleinste synchrone Intervall SSI liegt zwischen to und t4. Wird es um die Zeit AVmin vermindert, so ergibt sich der Zeitpunkt t3; wird es um die Zeit AVmax vermindert, so ergibt sich der Zeitpunkt t2. Demgemäß kann der in der Zeichnung dargestellte zeitliche Verlauf in vier Intervalle I bis IV unterteilt werden.

Während des normalen Betriebes mit veränderlicher Impulsrate (DDD-Betrieb) hat die AV-Verzögerung den Wert zwischen den Zeitpunkten t0 und t1. Wenn sich der Schrittmacher der höchsten synchronen Impulsrate nähert, bleibt die AV-Verzögerung konstant, bis das Intervall zwischen zwei natürlichen atrialen Aktivitäten (PP-Intervall) gleich der Differenz zwischen dem höchsten synchronen Intervall SSI (t4 - t0) und der kleinsten AV-Verzögerung ist (SSI - AVmin). Für PP-Intervalle in dem Bereich zwischen SSI - AVmax und SSI - AVmin bleibt das ventrikuläre Stimulationsintervall (VV-Intervall) konstant und ist gleich dem kleinsten synchronen Intervall (SSI). Unter diesen Umständen zeigt der Herzschrittmacher das typische Wenckebach-Verhalten der Veränderung der AV-Verzögerung bei konstantem VV-Intervall.

Für diejenigen Intervalle zwischen einem Stimulationsimpuls und der nächsten P-Welle, die kleiner als SSI - AVmax AVmax sind, erfolgt keine ventrikuläre Stimulation.

Zusammenfassend kann hinsichtlich der Zeichnung folgendes festgehalten werden:
Auftreten eines Vorhofsignales in

Bereich I keine ventrikuläre Stimulation
Bereich II ventrikuläre Stimulation bei t4 = SSI
Bereiche III, IV normaler DDD-Betrieb

Bei dem Ausführungsbeispiel gemäß Figur 2 liegen an einer Klemme 1 Vorhofsignale und an einer Klemme 2 Ventrikelsignale. Ein ventrikulärer Detektor 3 und eine Ausgangsstufe 4 geben bei jeder Detektion und Stimulierung einen Puls ab, der über ein ODER-Gatter 5 einen Zeitrechner 6 von einem Anfangswert aus startet. Das Ausgangssignal des Zeitrechners 6, la, ist eine logische Eins, wenn der Zeitrechner 6 sich in der Region I (Fig. 1) befindet. Das Signal wird in einer Stufe 7 invertiert und dann zu einem UND-Gatter 8 weitergeleitet. Das UND-Gatter 8 verhindert, daß atriale Detektionspulse, die durch einen Detektor 9 erfaßt werden, während der Region I den AV-Block 10 erreichen. Jedes atriale Signal bewirkt über eine Ausgangsstufe 11 und ein ODER-Gatter 12, daß ein AV-Intervall gestartet wird.

Das Ausgangssignal des AV-Blockes 10 setzt einen Flip-Flop 13, dessen Ausgangssignal in diesem Fall eine logische Eins ist, die einem UND-Gatter 14 zugeführt wird, wenn das AV-Intervall zu Ende ist.

Das Ausgangssignal des UND-Gatter 14 setzt das Flip-Flop 13 zurück und erzeugt einen ventrikulären Stimulationsimpuls über die Ausgangsstufe 4.

Dasselbe Signal, das den Zeitrechner 6 startet, startet auch einen SSI-Rechner 15. Das Ausgangssignal des SSI-Rechners 15 ist eine logische Null während des gesamten SSI-Intervalles und geht dann in eine logische Eins über.

Auf diese Weise bekommt man die übliche Wenckebach-Funktion.

**Patentansprüche**

1. Vorhofgesteuerter Herzschrittmacher, **dadurch gekennzeichnet**, daß ein kleinstes synchrones Intervall (SSI) zwischen zwei Stimulationsimpulsen (V) sowie eine minimale und eine maximale atrioventrikuläre Verzögerungszeit (AVmin, AVmax) vorgegeben sind, und daß eine Steuerschaltung (Fig. 2) vorhanden ist, die in Abhängigkeit vom Auftreten eines Vorhofsignales (A) bewirkt, daß der Herzschrittmacher mit veränderlicher Impulsrate arbeitet (DDD-Betrieb), wenn das Vorhofsignal (A) nach dem Zeitpunkt SSI - AVmin auftritt, daß der Herzschrittmacher mit der höchsten synchronen Impulsrate betrieben wird, wenn das Vorhofsignal (A) zwischen diesem Zeitpunkt SSI - AVmin und dem Zeitpunkt SSI - AVmax liegt und daß keine Stimulation erfolgt, wenn das Vorhofsignal (A) vor dem zuletzt genannten Zeitpunkt (SSI - AVmax) auftritt.

**Claim**

1. Auricle-controlled heart pacemaker, characterised in that a minimum synchronous interval (SSI) between two stimulation impulses (V) is selected, and a minimal and a maximal atrioventricular delay time (AVmin, AVmax) are selected, and in that there is a control circuit (Figure 2) which, in dependence upon the occurrence of an auricular signal (A), causes the heart pacemaker to operate with variable impulse rate (DDD operation) when the auricular signal (A) occurs after the time SSI - AVmin, and causes the heart pacemaker to be operated with the highest synchronous impulse rate when the auricular signal (A) occurs between the time SSI - AVmin and the time SSI - AVmax, and causes no stimulation to result when the auricular signal (A) occurs before the last-mentioned time (SSI–AVmax).

**Revendication**

1. Stimulateur cardiaque commandé par voie auriculaire, caractérisé par le fait qu'un intervalle synchrone extrêmement faible (SSI) entre deux impulsions de stimulation (V) ainsi que des retards atrioventriculaires minimum et maximum (AVmin, AVmax) sont prédéterminés et qu'il est prévu un circuit de commande (figure 2), qui agit en fonction de l'apparition d'un signal auriculaire (A) de telle sorte que le stimulateur cardiaque opère avec une fréquence d'impulsions variable (fonctionnement DDD) lorsque le signal auriculaire (A) apparaît après l'instant SSI-AVmin, que le stimulateur cardiaque opère avec la fréquence synchrone maximale d'impulsions lorsque le signal auriculaire (A) se situe entre cet instant SSI-AVmin et l'instant SSI-AVmax et au'aucune stimulation n'est exécutée lorsque le signal auriculaire (A) apparaît avant l'instant indiqué en dernier lieu (SSI-AVmax).

FIG 2

t1 = -AV    t0    t2 = SSI - AVmax    t4 = SSI

t3 = SSI - AVmin

FIG 1